# EUROPEAN PATENT APPLICATION

(11) **EP 4 606 908 A1**
(43) Date of publication of application: **27.08.2025**
(21) Application number: 24158803.7
(22) Date of filing: 21.02.2024
(51) Int. Cl.: C12Q 1/6809, C12Q 1/6823

(54) **METHOD OF INCREASING THE NUMBER OF USABLE CHANNELS IN MULTIPLEX PCR**

(71) Applicant: Roche Diagnostics GmbH, 68305 Mannheim (DE); F. Hoffmann-La Roche AG, 4070 Basel (CH)
(72) Inventor: Knobel, Rolf, 6343 Rotkreuz (CH)
(74) Representative: Altmann Stößel Dick Patentanwälte PartG mbB

(57) **Abstract**

The present invention relates to a method for performing a multiplex PCR to determine at least a first target polynucleotide and a second target polynucleotide in a sample, comprising (i) contacting said sample in a single container with at least a first probe oligonucleotide for the first target polynucleotide and with a second probe oligonucleotide for the second target polynucleotide; wherein said first probe oligonucleotide is labeled with a first label (label-1) emitting a label-1 signal in a first channel, a first label-1 crosstalk signal in a second channel, and a second label-1 crosstalk signal in a third channel, and wherein said second probe oligonucleotide is labeled with a second label (label-2) emitting a label-2 signal in said third channel; (ii) amplifying said target polynucleotides by PCR; (iii) measuring a multitude of values in the second channel and the third channel over at least part of the amplification in step (ii); and (iv) determining the first target polynucleotide based on the values measured in step (iii). The present invention also relates to systems, computer-readable storage media, and uses related to said method.

## Description

The present invention relates to a method for performing a multiplex PCR to determine at least a first target polynucleotide and a second target polynucleotide in a sample, comprising (i) contacting said sample in a single container with at least a first probe oligonucleotide for the first target polynucleotide and with a second probe oligonucleotide for the second target polynucleotide; wherein said first probe oligonucleotide is labeled with a first label (label-1) emitting a label-1 signal in a first channel, a first label-1 crosstalk signal in a second channel, and a second label-1 crosstalk signal in a third channel, and wherein said second probe oligonucleotide is labeled with a second label (label-2) emitting a label-2 signal in said third channel; (ii) amplifying said target polynucleotides by PCR; (iii) measuring a multitude of values in the second channel and the third channel over at least part of the amplification in step (ii); and (iv) determining the first target polynucleotide based on the values measured in step (iii). The present invention also relates to systems, computer-readable storage media, and uses related to said method.

Polymerase chain reaction (PCR) has long been a method of choice for biomedical analytic applications, in particular because of its specificity and sensitivity, but also its versatility. To reduce costs, PCR assays amplifying and detecting a multitude of target polynucleotides (multiplex-PCRs) have been developed. The extent of possible multiplexing is, however, limited by the available number of detection channels which can be reliably separated to avoid errors from signal crosstalk. E.g. if using fluorescent labels, typically only six or seven labels can be used without having significant overlap interference; in practice, even less channels may be possible.

This number of possible channels has been amplified by using a combination of color and temperature channels, as described e.g. in US 2018/0073064 A1, which may also be referred to as "TAGS-PCR". In this method, probe oligonucleotides for different target polynucleotides are labeled by different dyes, i.e. using more than one color channel, thus enabling differentiation of the respective amplification products. In addition, a multitude of probe oligonucleotides carrying the same label is differentiated by the melting points of tags they comprise. Thus, more than one temperature channel may be used per color channel.

By using TAGS-PCR, it is possible to determine a multitude of target polynucleotides; e.g. if five color channels and three temperature channels are used, theoretically a multiplex PCR with up to 15 detection channels is possible. In practice, however, it is not always possible to reliably compensate all channels against each other, so some channels are not usable, reducing the number of practically usable channels. This may be the case e.g. when the proportionality of signal bleeding over is not given, e.g. by continuing PCR reaction (elongation) between measurements.

Thus, there is still a need in the art for means and methods improving the number of practically usable channels in multiplex PCR, in particular in TAGS-PCR. This problem is addressed by the methods, systems, computer programs, and uses with the features of the independent claims. Advantageous embodiments which might be realized in an isolated fashion or in any arbitrary combinations are listed in the dependent claims.

In accordance, the present invention relates to a method for performing a multiplex PCR to determine at least a first target polynucleotide and a second target polynucleotide in a sample, comprising
(i) contacting said sample in a single container with at least a first probe oligonucleotide for the first target polynucleotide and with a second probe oligonucleotide for the second target polynucleotide;
   wherein said first probe oligonucleotide is labeled with a first label (label-1) emitting a label-1 signal in a first channel, a first label-1 crosstalk signal in a second channel, and a second label-1 crosstalk signal in a third channel, and
   wherein said second probe oligonucleotide is labeled with a second label (label-2) emitting a label-2 signal in said third channel;
(ii) amplifying said target polynucleotides by PCR;
(iii) measuring a multitude of values in the second channel and the third channel over at least part of the amplification in step (ii); and
(iv) determining the first target polynucleotide based on the values measured in step (iii).

In general, terms used herein are to be given their ordinary and customary meaning to a person of ordinary skill in the art and, unless indicated otherwise, are not to be limited to a special or customized meaning. As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements. Also, as is understood by the skilled person, the expressions "comprising a" and "comprising an" in an embodiment refer to "comprising one or more", i.e. are equivalent to "comprising at least one". In accordance, expressions relating to one item of a plurality, unless otherwise indicated, in an embodiment relate to at least one such item, in a further embodiment a plurality thereof; thus, e.g. identifying "a cell" relates to identifying at least one cell, in an embodiment to identifying a multitude of cells.

Further, as used in the following, the terms "preferably", "more preferably", "most preferably", "particularly", "more particularly", "specifically", "more specifically" or similar terms are used in conjunction with optional features, without restricting further possibilities. Thus, features introduced by these terms are optional features and are not intended to restrict the scope of the claims in any way. The invention may, as the skilled person will recognize, be performed by using alternative features. Similarly, features introduced by "in an embodiment" or similar expressions are intended to be optional features, without any restriction regarding further embodiments of the invention, without any restrictions regarding the scope of the invention and without any restriction regarding the possibility of combining the features introduced in such way with other optional or non-optional features of the invention.

The methods specified herein below, in an embodiment, are in vitro methods. The method steps may, in principle, be performed in any arbitrary sequence deemed suitable by the skilled person, but in an embodiment are performed in the indicated sequence; also, one or more, in an embodiment all, of said steps may be assisted or performed by automated equipment. Moreover, the methods may comprise steps in addition to those explicitly mentioned above. Furthermore, the terms "first", "second", "third" and the like in the description and in the claims are used for distinguishing between non-identical elements falling under the same designation and not necessarily for describing a sequential or chronological order or an order of preference.

As used herein, if not otherwise indicated, the term "about" relates to the indicated value with the commonly accepted technical precision in the relevant field, in an embodiment relates to the indicated value ± 20%, in a further embodiment ± 10%, in a further embodiment ± 5%. Further, the term "essentially" indicates that deviations having influence on the indicated result or use are absent, i.e. potential deviations do not cause the indicated result to deviate by more than ± 20%, in a further embodiment ± 10%, in a further embodiment ± 5%. Thus, "consisting essentially of" means including the components specified but excluding other components except for materials present as impurities, unavoidable materials present as a result of processes used to provide the components, and components added for a purpose other than achieving the technical effect of the invention. For example, a composition defined using the phrase "consisting essentially of" encompasses any known acceptable additive, excipient, diluent, carrier, and the like. In an embodiment, a composition consisting essentially of a set of components will comprise less than 5% by weight, in a further embodiment less than 3% by weight, in a further embodiment less than 1% by weight, in a further embodiment less than 0.1% by weight of non-specified component(s).

The term "polynucleotide", as used herein, refers to a linear or circular nucleic acid molecule. The polynucleotide of the present invention shall be provided, preferably, either as an isolated polynucleotide (i.e. isolated from its natural context) or in genetically modified form, in an embodiment comprising at least one heterologous sequence. The term polynucleotide encompasses single- as well as, partially or completely, double-stranded polynucleotides. In an embodiment, the polynucleotide is a DNA polynucleotide, which may also be referred to as "DNA". Moreover, comprised are also chemically modified polynucleotides including naturally occurring modified polynucleotides such as glycosylated or methylated polynucleotides or artificially modified derivatives such as biotinylated polynucleotides, locked nucleic acids, peptide nucleic acids, and the like. In an embodiment, the nucleotide modification is independently selected from the group consisting of a Locked Nucleic Acid (LNA) nucleotide, a Peptide Nucleic Acid (PNA) nucleotide, a Bridged Nucleic Acid (BNA) nucleotide, a 2'-O alkyl substitution, an L-enantiomeric nucleotide, and combinations thereof. In view of the description herein, template DNA, e.g. for PCR, primers, e.g. sequencing or amplification primers, and probe oligonucleotides are included in the term polynucleotides.

The polynucleotide may be a target polynucleotide, the term "target polynucleotide" being used in its conventional meaning known to the skilled person. Preferably, the target polynucleotide is a polynucleotide for which it is desirable that it shall be determined. Thus, the target polynucleotide may e.g. be a diagnostic polynucleotide which is indicative is a bodily condition, in an embodiment a disease. Thus, the target polynucleotide in an embodiment is known or suspected to be comprised in a sample of a subject. The target molecule may, however, also e.g. be a polynucleotide indicative of the presence of a biological material, e.g. in an environmental sample. Thus, the target polynucleotide may be a polynucleotide of a living organism, in an embodiment an infectious agent, in a further embodiment of a virus, a bacterium, or a eukaryotic infectious agent. In an embodiment, the target polynucleotide is a polynucleotide of a subject, in an embodiment comprising a nucleic acid sequence indicative of a bodily state of said subject. Thus, the target polynucleotide may comprise a nucleic acid sequence indicative of a hereditary disease and/or of a somatic mutation. In particular, the target polynucleotide may comprise a mutation, e.g. may comprise a nucleic acid sequence encoding a cancer antigen, in an embodiment a cancer specific antigen.

The polynucleotide may be an oligonucleotide. In concurrence with common general understanding of the skilled person, polynucleotides comprising at most 30 consecutive nucleotide may be referred to as "oligonucleotides" for clarity, while polynucleotides comprising more than 30 consecutive nucleotides are typically referred to as "polynucleotides".

The oligonucleotide may in particular be a primer oligonucleotide or a probe oligonucleotide. The term "primer oligonucleotide" is known to the skilled person and, in an embodiment, relates to an at least partially single-stranded oligonucleotide specifically hybridizing to a target polynucleotide and providing a start point for elongation of polynucleotide biosynthesis by a thermostable DNA polymerase. As the skilled person understands, in PCR, in particular real-time PCT, typically a pair of primer oligonucleotides is used to amplify a target polynucleotide or a fragment, i.e. a subsequence, thereof. A "probe oligonucleotide" is, in principle, also known to the skilled person. As referred to herein, a probe oligonucleotide in an embodiment comprises an at least partially single-stranded oligonucleotide specifically hybridizing to a target polynucleotide or fragment thereof that can in an embodiment be amplified by using a pair of primer oligonucleotides. In real-time PCR, the probe oligonucleotide in an embodiment comprises at least one indicator and at least one quencher. The principles underlying real-time probe oligonucleotide design are known to the skilled person. Typically, the indicator and the quencher are comprised towards the ends of the probe oligonucleotide, however, any other spatial arrangement deemed appropriate by the skilled person may be used, in an embodiment provided that in the intact free, i.e. non-hybridized, probe oligonucleotide and in the hybridized probe oligonucleotide, the quencher significantly reduces the intensity of the optical signal generated by the indicator, and that the quencher can be separated from the indicator by a 5'->3' exonuclease activity of a thermostable DNA polymerase. Thermostable DNA polymerases having the aforesaid exonuclease activity are known in the art, e.g. Thermus aquaticus DNA polymerase (Taq polymerase). Thus, the indicator and the quencher in the probe polynucleotide are intervened by at least one nuclease-susceptible cleavage site.

In multi-temperature channel real-time PCR, the probe oligonucleotide, which may also be referred to as "multi-temperature probe oligonucleotide", at least one, in an embodiment two, probe oligonucleotides may in particular have the structure described in US 2018/0073064 A1; probe oligonucleotides having this structure are also referred to as "multi-temperature probe oligonucleotides". However, in an embodiment, the lowest temperature channel probe oligonucleotide typically does not have the structure of a multi-temperature probe oligonucleotide, but instead is a standard real-time PCR probe oligonucleotide comprising an indicator and a quencher, but typically no tag portion.

In an embodiment, the multi-temperature probe oligonucleotide comprises an annealing portion and a tag portion. In an embodiment, the tag portion is attached to the 5' terminus of the annealing portion . In another embodiment, the tag portion is attached to the 3' terminus of the annealing portion . In yet another embodiment, the tag portion is attached via a linker to a region of the annealing portion. In an embodiment, an indicator is located on the tag portion, an annealing portion quencher is located on the annealing portion, and a tag portion quencher is located on the tag portion. In such case, in an embodiment, the tag portion is covalently connected to the annealing portion such that the tag portion can be separated from the annealing portion by a 5'->3' exonuclease activity of a thermostable DNA polymerase, i.e. the indicator on the tag portion and the annealing portion quencher are intervened by at least one nuclease-susceptible cleavage site. In accordance, the aforesaid separation of the tag portion from the annealing portion can be put into practice by a thermostable DNA polymerase degrading an annealing portion annealed to a target polynucleotide or to a fragment thereof during the elongation step of PCR. As will be understood by the skilled person, the aforesaid separation of the tag portion from the annealing portion stops quenching of the indicator in the tag portion by the annealing portion quencher. Also in an embodiment, the indicator and the tag portion quencher are located on different strands of the tag portion and can be separated by increasing temperature beyond the melting temperature of the tag portion. Thus, during multi-temperature channel real-time PCR, the indicator in the multi-temperature probe oligonucleotide initially is quenched by the annealing portion quencher and by the tag portion quencher. When temperature is increased, e.g. during temperature-channel measuring steps, the tag portion may dissociate into single strands such that the tag portion quencher is separated from the indicator in the tag portion; however, the signal of the indicator is still quenched by the annealing portion quencher. In the converse, during the elongation step of PCR, in case the target polynucleotide or a fragment thereof is amplified, the annealing portion quencher may become separated from the indicator by the activity of the thermostable DNA polymerase, but the indicator is still quenched by the tag portion quencher. Thus, only in case the annealing portion was cleaved off the tag portion, which is indicative of the presence of the target polynucleotide, said cleavage can be detected by increasing the temperature beyond the melting temperature of the tag portion. By using tag portions having different melting points, detection of different target polynucleotides in different temperature channels can be put into practice.

Thus, the annealing portion of the multi-temperature probe oligonucleotide in an embodiment comprises a nucleotide sequence at least partially complementary to the target polynucleotide and hybridizes to a region of the target polynucleotide that is bounded by the pair of primer oligonucleotides used to amplify the target oligonucleotide or fragment thereof. The annealing portion further comprises at least one quencher, in an embodiment attached to the annealing portion such that the signal of the indicator in the tag portion is quenched. Thus, the quencher may in particular be attached in close proximity, in an embodiment within one nucleotide, in a further embodiment within two nucleotides, in a further embodiment within three nucleotides, in a further embodiment within four nucleotides, of the attachment site of the tag portion. Thus, the annealing portion may essentially correspond to a standard probe oligonucleotide of a real-time PCR lacking an indicator.

The aforesaid tag portion in an embodiment comprises an at least partially double-stranded oligonucleotide with a nucleotide sequence non-complementary to the target polynucleotide, in an embodiment non-complementary to any target polynucleotide to be detected in a given PCR. In one embodiment, the tag portion of the probe oligonucleotide comprises an indicator attached to a first strand of the at least partially double-stranded oligonucleotide, wherein said first strand is covalently connected to the annealing portion of the multi-temperature probe oligonucleotide, and comprises a quencher attached to a second strand of the at least partially double-stranded oligonucleotide. In particular the tag portion, but also the annealing portion, may comprise at least one nucleotide modification, wherein the at least one nucleotide modification in an embodiment is selected from the group consisting of Locked Nucleic Acid (LNA) , Peptide Nucleic Acid (PNA) , Bridged Nucleic Acid (BNA) , 2'-O alkyl substitution, L-enantiomeric nucleotide, and a combination of at least two of the aforesaid. Also, the tag portion in an embodiment comprises a modification such that it is not capable of being extended by a thermostable DNA polymerase. As described herein above, in case of multi-temperature channel real-time PCR, the tag portions of different multi-temperature probe oligonucleotides in an embodiment have different melting points. The skilled person knows means and methods for adjusting the melting point of double-stranded oligonucleotides, e.g. by increasing the number of complementary nucleotides, using modified nucleotides, e.g. LNA nucleotides, and the like.

As the skilled person is aware of, specific hybridization in an embodiment is accomplished by providing a polynucleotide, in an embodiment an oligonucleotide, which is essentially complementary to the target polynucleotide over a stretch of at least 10, in an embodiment at least 15, in a further embodiment at least 20, contiguous nucleotides, in an embodiment contiguous 3' nucleotides. Said stretch of nucleotides is, in an embodiment, 85%, in a further embodiment 90%, in a further embodiment 95%, in a further embodiment 99%, in a further embodiment 100% identical to a sequence region comprised by a target polynucleotide.

Unless specifically indicated otherwise, reference to specific polynucleotides herein in an embodiment includes polynucleotide variants. The term "polynucleotide variant", as used herein, relates to a variant of a polynucleotide referred to herein comprising a nucleic acid sequence characterized in that the sequence can be derived from the aforementioned specific nucleic acid sequence by at least one nucleotide substitution, addition and/or deletion, wherein the polynucleotide variant shall have the function and/or activity as specified for the specific polynucleotide. Thus, a variant of a polynucleotide may e.g. be an ortholog, a paralog, or another homolog of the specific polynucleotide; the polynucleotide variant may also be a mutant of the specific polynucleotide, in an embodiment a naturally occurring mutant, e.g. identified in a cancer cell. Also in an embodiment, said polynucleotide variant is or is derived from a non-naturally occurring allele of the specific polynucleotide. Further polynucleotide variants include polynucleotides comprising nucleic acid sequences which are at least 70%, in an embodiment at least 80%, in a further embodiment at least 90%, even in a further embodiment at least 95%, still in a further embodiment at least 98%, most in an embodiment at least 99%, identical to the specifically indicated nucleic acid sequences. The percent identity values are, in an embodiment, calculated over the entire nucleic acid sequence region, in an embodiment as specified herein elsewhere. The polynucleotides of the present invention either consist, essentially consist of, or comprise the aforementioned nucleic acid sequences. Thus, they may contain further nucleic acid sequences as well.

The degree of identity (e.g. expressed as "%identity") between two biological sequences, in an embodiment DNA, RNA, or amino acid sequences, can be determined by algorithms well known in the art. In an embodiment, the degree of identity is determined by comparing two optimally aligned sequences over a comparison window, where the fragment of sequence in the comparison window may comprise additions or deletions (e.g., gaps or overhangs) as compared to the sequence it is compared to for optimal alignment. The percentage is calculated by determining, in an embodiment over the whole length of the polynucleotide or polypeptide, the number of positions at which the identical residue occurs in both sequences to yield the number of matched positions, dividing the number of matched positions by the total number of positions in the window of comparison and multiplying the result by 100 to yield the percentage of sequence identity. Optimal alignment of sequences for comparison may be conducted by the local homology algorithm of Smith and Waterman (1981), by the homology alignment algorithm of Needleman and Wunsch (1970), by the search for similarity method of Pearson and Lipman (1988), by computerized implementations of these algorithms (GAP, BESTFIT, BLAST, PASTA, and TFASTA in the Wisconsin Genetics Software Package, Genetics Computer Group (GCG), 575 Science Dr., Madison, WI), or by visual inspection. Given that two sequences have been identified for comparison, GAP and BESTFIT are in an embodiment employed to determine their optimal alignment and, thus, the degree of identity. In an embodiment, the default values of 5.00 for gap weight and 0.30 for gap weight length are used. In the context of biological sequences referred to herein, the term "essentially identical" indicates a %identity value of at least 80%, in an embodiment at least 90%, in a further embodiment at least 98%, in a further embodiment at least 99%. As will be understood, the term essentially identical includes 100% identity. The aforesaid applies to the term "essentially complementary" mutatis mutandis.

The term "fragment" of a biological macromolecule, in an embodiment of a polynucleotide, is used herein in a wide sense relating to any sub-part of the respective biological macromolecule comprising the indicated sequence, structure and/or function. Thus, the term includes sub-parts generated by actual fragmentation of a biological macromolecule, but also sub-parts derived from the respective biological macromolecule in an abstract manner, e.g. in silico.

Unless specifically indicated otherwise herein, the compounds specified, in particular the polynucleotides, may be comprised in larger structures, e.g. may be covalently or non-covalently linked to further sequences, indicators, or other compounds deemed appropriate by the skilled person.

The term "polymerase chain reaction", abbreviated as "PCR" is known to the skilled person. The term "multiplex PCR", as used herein, relates to each and every PCR method adapted for the detection of more than one target polynucleotide. Also, the term "real-time PCR" relates to each and every PCR adapted to measure at least one signal correlating with the amount of amplification product(s) during amplification, e.g. at least once per amplification cycle for at least ten, in an embodiment at least 20, in a further embodiment at least 30, in a further embodiment for all amplification cycles. In an embodiment, the multiplex PCT is a multi-color channel multiplex PCR, i.e. is a PCR in which different indicators are used to differentiate between non-identical probe oligonucleotides. In a further embodiment, the multiplex PCR is a multi-temperature channel multiplex PCR, i.e. is a PCR in which non-identical probe oligonucleotides are differentiated by their melting points. Multi-temperature channel multiplex PCR has been described herein above and e.g. in US 2018/0073064 A1. Thus, in an embodiment, the multiplex PCR is multiplexed real-time PCR for detection and quantitation of target nucleic acids using tagged hydrolysis probes as described in US 2018/0073064 A1. In a further embodiment, the multiplex PCR is a combined multi-color channel multi-temperature channel multiplex PCR, i.e. a PCR using at least two non-identical indicators in non-identical color channels, and using at least two non-identical temperature channels for at least one of said color channels.

As used herein, the term to "determine" is understood by the skilled person and in an embodiment refers to establishing data indicative of the presence or absence of a target polynucleotide and, if present, in an embodiment, the concentration or amount of the target polynucleotide. The target polynucleotide in an embodiment is determined by hybridizing a probe oligonucleotide to said target polynucleotide or to a PCR product thereof, in a further embodiment based on a signal generated by an indicator comprised by the probe oligonucleotide as specified herein elsewhere. In an embodiment, determining a target polynucleotide comprises establishing whether a target polynucleotide is present or absent in the sample at a concentration above the detection limit of the method, i.e., in an embodiment, the determining is qualitative. Methods of determining a detection limit are known to the skilled person. In a further embodiment, determining is determining semi-quantitatively or quantitatively the amount or concentration of a target polynucleotide in a sample. For semiquantitative determining, the amount may be assigned e.g. to two or more pre-defined categories, e.g. above or not above a reference value, or low, medium, or high. For quantitative determination, either the absolute or precise amount and/or concentration of the target polynucleotide may be determined, or the relative amount of the target polynucleotide may be determined. The relative amount may be determined in a case were the precise amount of a target polynucleotide can or shall not be determined. In such case, it can be determined whether the amount in which the target polynucleotide is present is increased or diminished with respect to a reference sample comprising said target polynucleotide in a pre-determined amount. For quantitative determination, any parameter correlating with the amount or concentration of the target polynucleotide in the sample or any value derived therefrom by standard mathematical and/or evaluation operations, including in particular multiplication, division, reciprocal formation, scaling, normalization, standardization, error correction, background correction, or mean or median calculation, may be determined and/or output.

Methods of determining if an amplified polynucleotide is present or absent and optionally for its quantification are well known in the art and preferably rely on the comparison of the amount and/or identity of the amplified polynucleotides to suitable controls, as exemplified in the Examples described herein below. If an amplified polynucleotide that is specific for an individual target polynucleotide is present, in an embodiment above background levels, then the respective target polynucleotide is present in the sample and may, optionally, be quantified. If there is no amplification product for a polynucleotide that is specific for a certain target polynucleotide and, thus, if an amplified polynucleotide is absent, the respective target polynucleotide is not present in the sample.

In an embodiment, a multitude of target polynucleotides is determined and thereby detected simultaneously. The determination of target polynucleotides in an embodiment is done by identifying amplified polynucleotides that are specific for the target polynucleotide to be detected. In an embodiment, the amplification of polynucleotides that are specific for the respective target polynucleotide is done in only one container, thus, in the same container. A typical container is a reaction tube or a well in a multi-well plate. Such containers are well known in the art and, e.g., are commonly as referred to as PCR tubes or wells of a PCR multi-well plate. As the skilled person understands, the determination may also be performed in any other format deemed appropriate by the skilled person, e.g. as a droplet PCR. It is thus to be understood that the form of the container may depend on the specific PCR setup used.

The term "amplifying " is known to the skilled person; in an embodiment, amplifying relates to any measure causing the concentration of a target polynucleotide or a fragment thereof to increase. In an embodiment, the term refers to a step of submitting an amplification solution comprising or suspected to comprise a target polynucleotide to conditions causing amplification of the target polynucleotide or a fragment thereof in case said target polynucleotide is present in said solution. Components of an amplification solution may include, but are not limited to, e.g., at least one or a pair of primer oligonucleotide(s), a polynucleotide template, e.g. from a sample, a thermostable DNA polymerase, nucleotides, dNTPs, and/or any further compound(s) deemed appropriate by the skilled person. In an embodiment, amplifying is exponentially amplifying; however, amplifying may also be linear, or any increase between linear and exponential amplification, e.g. in case of suboptimal amplification, or in case only one primer oligonucleotide is included in the amplification solution. Establishing a suitable PCR program for amplifying a target polynucleotide of interest is a standard proceeding for the skilled person.

The term "channel" is used herein in its common general meaning assigned to it in the given technical context. In an embodiment, the channel is any, typically artificially allocated, subsection of a parameter range used for determining an analyte. Thus, the channel may e.g. be a subrange of a temperature range, of a color range, of a fluorescence spectrum, and the like. In an embodiment, the channel is a subrange of a temperature range. In an embodiment, the multiplex PCR is a multi-temperature channel multiplex PCR and a first channel is a first temperature, a second channel is a second temperature, and a third channel is a third temperature, wherein said first, second, and third temperatures in an embodiment are selected from predetermined non-overlapping temperature ranges. In an embodiment, said first temperature is lower than the second temperature and said second temperature is lower than the third temperature.

As the skilled person understands in view of the description herein, a "temperature" of a temperature channel may as well be a temperature range within one of the aforesaid pre-determined temperature ranges. Thus, a temperature may be the indicated temperature ±15%, in an embodiment ±10%, in a further embodiment ±5%, in a further embodiment ±2%, in a further embodiment ±1%. In an embodiment, the temperature of the temperature channel is the indicated temperature within the technical precision provided by the device used, i.e. is the indicated temperature ±1°C, in an embodiment ±0.5°C, in a further embodiment ±0.2°C. In an embodiment, the temperature of the first channel is more than 40°C and up to 65°C, the temperature of the second channel is more than 65°C and up to 80°C, and/or the temperature of the third channel is more than 80°C and up to 100°C. In the case of temperature channels, the channels in an embodiment are separated such that signals can be optimally, but typically not completely, separated between the channels. Thus, in an embodiment, the temperature difference between the first channel and the second channel is at least 10°C, in an embodiment is at least 15°C, and/or the temperature difference between the second channel and the third channel is at least 10°C, in an embodiment is at least 15°C.

Nonetheless, despite optimization of channel separation, crosstalk signalling cannot be avoided in practice, so signals from a given channel may require compensation for crosstalk signals from one or more other channels. The term "crosstalk signal" is known to the skilled person and methods for compensation of crosstalk signals are, in principle, known in the art. In principle, to determine crosstalk signals, a detection reaction may be performed for a single channel, however, additionally measuring values in at least one further, e.g. the neighboring channel. Mathematical methods for compensation crosstalk signals are known in the art and comprise, in an embodiment, linear correction of the values measured for a channel for crosstalk signal values measured in at least one further channel. In an embodiment, a crosstalk signal is a signal caused by a detection process in a first channel, but measured in a second channel. E.g., in multi-temperature channel PCR, detection in the first temperature channel causes unquenching of the indicator comprised therein. If temperature is increased to the temperature of the second channel, the signal caused by the indicator of the first probe oligonucleotide remains detectable and therefore must be compensated to obtain the (additional) signal caused by the second probe oligonucleotide.

As referred to herein, the term "indicator moiety", which may also be referred to as "indicator", relates to any substructure of a probe oligonucleotide providing a detectable signal. The detectable signal may be any signal deemed detectable by the skilled person. In an embodiment, the detectable feature is a physical feature, in an embodiment selected from the consisting of fluorescence, absorption, transmission, color, reflection, and radiation. In a further embodiment, the detectable signal is a fluorescence. Thus, the indicator may be a fluorescent dye, in an embodiment a quenchable fluorescent dye. Corresponding dyes are known in the art, and include in particular dyes like Cy5.5 (CAS No.210892-23-2, 1H-Benz[e]indolium, 2-[5-[3-(5-carboxypentyl)-1,3-dihydro-1,1-dimethyl-6,8-disulfo-2H-benz[e]indol-2-ylidene]-1,3-pentadien-1-yl]-3-ethyl-1,1-dimethyl-6,8-disulfo-), 6-Carboxyfluorescein (6-FAM; FAM)), 6-carboxy-2',4,4',5',7,7'-hexachlorofluorescein (6-HEX, HEX), and JA270. In case the multiplex PCR is a multi-temperature channel PCR, the indicator may be the same for the first and second probe oligonucleotide, in an embodiment for all probe oligonucleotides.

The term "quencher moiety", which may also be referred to as "quencher", is known to the skilled person. In an embodiment, the term relates to any substructure of a probe oligonucleotide causing an emission of an indicator to decrease in case said indicator is in close molecular proximity to the quencher. Appropriate quenchers for pre-determined indicators are known in the art and are commercially available. E.g. Black Hole Quencher^{™}-2 (BHQ-2) or Black Hole Quencher^{™}-3 (BHQ-3) may be used if Cy5.5 is used as an indicator. In case the multiplex PCR is a multi-temperature channel PCR, the quencher may be the same for the first and second probe oligonucleotide, in an embodiment for all probe oligonucleotides. Also, the quencher may be the same moiety in the annealing portion quencher and the tag portion quencher.

The term "label", as referred to herein, relates to all components contributing to the detectable signal in a probe oligonucleotide. Thus, the label may be an indicator as specified herein above or may be a combination of an indicator and a quencher in a multiplex PCR probe oligonucleotide. In multi-temperature probe oligonucleotides, the label in an embodiment is the combination of at least two quenchers and at least one indicator as described herein above. As referred to herein, a label comprising two components which may interact and, as a consequence of said interaction, provide a modulated signal, is also referred to as "interactive label"; moreover, an interactive label comprising more than one interacting partner for the indicator, e.g. at least two quencher moieties, may also be referred to as an "interactive dual label". In an interactive dual label, a signal emitted by the indicator comprised in the label may depend on the state of the probe oligonucleotide; i.e. in case of an intact annealing portion and/or a double stranded tag portion, the signal of the label may be low, while in case of cleavage of the annealing portion from the tag portion and dissociation of the two strands of the tag portion, the signal may be high. In case the multiplex PCR is a multi-temperature channel PCR, the label may be the same for the first and second probe oligonucleotide, in an embodiment for all probe oligonucleotides.

As referred to herein, a first probe oligonucleotide may comprise a first label, which may also be referred to as label-1. The label-1 emits a label-1 signal in a first channel, a first label-1 crosstalk signal in a second channel, and a second label-1 crosstalk signal in a third channel. In case of a multi-temperature probe oligonucleotide, the label may comprise an indicator moiety comprised by a first strand of the tag portion of the probe oligonucleotide, a tag portion quencher, and an annealing portion quencher, all as specified herein above, i.e. may be an interactive dual label as specified herein above. In accordance, the label-2 may emit the aforesaid signals only after double de-quenching, as specified herein above. As discussed herein above, the same may apply mutatis mutandis to the label-1; however, in an embodiment discussed herein above, the label-1 comprises only an indicator and a quencher, both covalently connected to the annealing portion, and does not comprise a tag portion.

The term "sample", as used herein, relates to a sample known or suspected to comprise at least one target polynucleotide. In an embodiment, the sample is or comprises a sample of a body fluid, a sample from a tissue or an organ, or a sample of wash/rinse fluid or a swab or smear obtained from an outer or inner body surface. Body fluid samples include samples of blood, plasma, serum, urine, saliva, and lacrimal fluid. Samples can be obtained by use of brushes, (cotton) swabs, spatula, rinse/wash fluids, punch biopsy devices, puncture of cavities with needles or lancets, or by surgical instrumentation. However, samples obtained by well-known techniques including, in an embodiment, scrapes, swabs or biopsies from the urogenital tract, perianal regions, anal canal, the oral cavity, the upper aerodigestive tract and the epidermis are also included as samples. Cell-free fluids may be obtained from the body fluids or the tissues or organs by lysing techniques such as homogenization and/or by separating techniques such as filtration or centrifugation. It is to be understood that the sample may be further processed in order to carry out the method. Particularly, cells may be removed from the sample by methods and means known in the art, calcium ions may be complexed to prevent coagulation, coagulation may be induced, and the like. Moreover, at least one analyte, e.g. DNA and/or RNA, may be enriched, extracted, and/or purified from the sample by methods and means known in the art, thus providing a template for multiplex PCR. Thus, the term sample also may relate to preparations comprising or suspected to comprise at least one target polynucleotide which are diluted, enriched, purified and/or extracted from a sample. In an embodiment, the sample is a sample not derived from a subject, e.g. a water sample, a sewage sample, a food sample, or the like.

The term "contacting", as used herein, is understood by the skilled person. In an embodiment, the term relates to bringing at least a first and a second probe oligonucleotide in physical contact with a sample and optionally with further compounds of a reaction solution and thereby, e.g. allowing the sample and the probe oligonucleotides to interact.

The term "measuring" is understood by the skilled person. In an embodiment, the term relates to establishing at least one value of a parameter to be measured. Means and measures for measuring values of the parameters referred to herein are in principle known to the skilled person. In case the label comprises or is an indicator emitting a fluorescent signal, the parameter to be measured in an embodiment is the intensity of said fluorescence, and values thereof may be measured. Values of parameters are measured in the channels as indicated, in an embodiment even though the indicator moiety may be the same for all channels, e.g. in multi-temperature multiplex PCR.

The method comprises step (i) contacting said sample in a single container with at least a first probe oligonucleotide for the first target polynucleotide and with a second probe oligonucleotide for the second target polynucleotide; wherein said first probe oligonucleotide is labeled with a first label (label-1) emitting a label-1 signal in a first channel, a first label-1 crosstalk signal in a second channel, and a second label-1 crosstalk signal in a third channel, and wherein said second probe oligonucleotide is labeled with a second label (label-2) emitting a label-2 signal in said third channel.

In multi-temperature channel PCR, the second probe oligonucleotide, and optionally the first probe oligonucleotide, in an embodiment are multi-temperature probe oligonucleotides as described herein above, i.e. said probe oligonucleotides comprise an annealing portion and a tag portion, and comprise a label comprising at least two quencher moieties and at least one indicator moiety, all as described herein above.

The method further comprises step (ii) amplifying at least one of said target polynucleotides by PCR. Appropriate PCR protocols, PCR primers, reaction solution compositions, and other parameters for performing step (ii) are selected by the skilled person for the specific application without further ado. As the skilled person understands in view of the description herein, step (ii) in an embodiment comprises repetition of the PCR steps of annealing, elongation, and denaturation, which are repeated so as to cause amplification of one or more target polynucleotide(s), if present, in the sample. If a target polynucleotide is present in the sample, the probe oligonucleotide binds to the target polynucleotide or an amplification product thereof, and the probe oligonucleotide is degraded by the 5'->3' exonuclease activity of the thermostable polymerase. In the case of a multi-temperature probe oligonucleotide, only the annealing portion is degraded in step (ii), leaving a double stranded tag portion in which the signal of the indicator is quenched by the tag portion quencher. Thus, depending on the amount of target polypeptide and its target product in the reaction container, a defined amount of tag portions will be liberated from multi-temperature probe oligonucleotides.

The method further comprises step (iii) measuring a multitude of values in the second channel and the third channel over at least part of the amplification in step (ii). Methods for measuring said signals have been described herein above. The signals may be measured after at least one amplification step of step (ii), e.g. after the last amplification step. In an embodiment, the signals are measured after each amplification step in step (ii). Thus, the result of step (ii) may e.g. be courses of changes of signals over amplification steps for the second and the third channel, which comprise the first label-1 crosstalk signal, the second label-1 crosstalk signal, and the label-2 signal.

In multi-temperature channel PCR, step (ii) in an embodiment comprises heating the reaction solution to the temperature of each of said temperature channels, in an embodiment after the elongation step in step (ii). E.g. in case of a three temperature channel PCR, the reaction solution in an embodiment is heated to the temperature of the first temperature channel, optionally followed by measuring the label-1 signal; then to the temperature of the second temperature channel followed by measuring the first label-1 crosstalk signal; then to the temperature of the third temperature channel, followed by measuring the second label-1 crosstalk signal and the label-2 signal.

The method further comprises step (iv) determining the first target polynucleotide based on the values measured in step (iii). Thus, in an embodiment, step (iv) is determining the first target polynucleotide based on the values of the first label-1 cross-talk signal (which is measured in the second channel) and determining the second target polynucleotide based on the values of the label-2 signal and the values of the second label-1 crosstalk signal (which are measure in the third channel) determined in step (iii).

The term determining has been discussed herein above. The expression determining a target polynucleotide "based on" one or more signals is understood by the skilled person. In an embodiment, said expression relates to determining the target polynucleotide only based on the indicated signal(s). Thus, in an embodiment, the first target polynucleotide is determined taking into account only values measured in the second channel, i.e. for the first label-1 crosstalk signal, and the second target polynucleotide is determined taking into account only values measured for the second label-1 crosstalk signal and for the label-2 signal. Thus, the first and second target polynucleotide in an embodiment are determined not taking into account values from the first channel, i.e. of the label-1 signal, i.e. the signal which would be used for determining the first target polynucleotide in standard multi-temperature channel PCR.

In an embodiment, crosstalk compensation between channels, which may also be referred to as crosstalk correction or "compensation", is performed according to standard methods known in the art.

Thus, in an embodiment, cross-talk signals are linearly compensated. As shown herein below in the Examples, however, linear compensation may not be possible for all channels. In particular, in a three temperature channel PCR, in an embodiment as described herein above, it was found that there is a non-linear cross-talk signal from the low-temperature channel to the medium-temperature channel, which cannot be linearly compensated, so in an embodiment the first label-1 crosstalk signal cannot be linearly compensated in step (iv). However, it was found that the second label-1 crosstalk signal can be linearly compensated in step (iv), so the method in an embodiment further comprises determining the second target polynucleotide based on the values of the label-2 signal and the values of the second label-1 crosstalk signal, in an embodiment by linearly compensating the label-2 signal for the second label-1 crosstalk signal. Thus, as referred to herein, the method comprises determining a first target polynucleotide in the given color channel by measuring the signal of label-1 in the medium-temperature channel instead of the low-temperature channel, and measuring a second target polynucleotide in the high-temperature channel, in an embodiment linearly compensating crossover signal from the low-temperature channel. In accordance, as referred to herein, the first label-1 crosstalk signal cannot be linearly compensated in step (iv). Thus, in an embodiment, step (iv) comprises linearly compensating the second label-1 crosstalk signal based on the label-1 signal when determining the second target polynucleotide.

In view of the above, the instant invention relates to a method for performing a multi-temperature multiplex PCR to determine at least a first target polynucleotide and a second target polynucleotide in a sample, comprising
(i) contacting said sample in a single container with at least a first probe oligonucleotide for the first target polynucleotide and with a second probe oligonucleotide for the second target polynucleotide;
   wherein said first probe oligonucleotide is labeled with a first label (label-1) emitting a label-1 signal in a first channel, a first label-1 crosstalk signal in a second channel, and a second label-1 crosstalk signal in a third channel, wherein said first, second, and third channels are temperature channels; and
   wherein said second probe oligonucleotide is labeled with a second label (label-2) emitting a label-2 signal in said third channel;
   wherein the label-1 and the label-2 comprise the same indicator moiety, wherein said indicator moiety in an embodiment comprises a cyanine dye, in an embodiment Cy5.5,
(ii) amplifying at least one of said target polynucleotides by PCR;
(iii) measuring a multitude of values in the second channel and the third channel over at least part of the amplification in step (ii); and
(iv) determining the first target polynucleotide based on the values determined in step (iii).

Advantageously, it was found in the work underlying the present invention that while it may not be possible to compensate a crosstalk signal linearly in a neighboring channel in a multiplex PCR, linear compensation in fact is possible of the label-2 signal for the second label-2 crosstalk signal. In other words, the signal of a first channel may be measured in its first crosstalk channel, while the possibility of linear compensation enables to use the third channel for determining an additional target polynucleotide.

The definitions made above apply mutatis mutandis to the following. Additional definitions and explanations made further below also apply for all embodiments described in this specification mutatis mutandis.

The present invention further relates to a system comprising at least one processor configured to
(I) obtain measurement data from step (iii) of a method described herein; and
(II) perform at least determining step (iv) of said method.

The term "obtaining", and grammatical variations thereof, as used herein, relate to acquiring the indicated information, in particular a value of a parameter such as measurement values in a channel at a multitude of time points, in a manner enabling basing the determining on said information. Thus, in an embodiment, obtaining is reading the information from a data carrier, e.g. in the form of a data sheet, analysis device output, e.g. a result of an multiplex PCR, or the like; or from a database comprising at least the relevant information. In an embodiment, the information obtained comprises measurement values determined as specified herein above. The data carrier and/or the database may be local, i.e. physically connected to the system referred to herein; they may, however, also be remote, accessible via a network connection or via the internet; thus, the data carrier may e.g. be cloud storage. Also, the steps performed by the system may be implemented as a service provided by means of a network connection, e.g. via internet, in which measurement values are obtained from e.g. a device performing the determination and the result of the determining is output.

The term "system", as used herein, relates to a system of means comprising at least the aforementioned means operatively linked to each other as to allow the determination. Typical means for obtaining measurement data and means for carrying out the determination are disclosed herein above in connection with the methods of the invention. How to link the means in an operating manner will depend on the type of means included into the system. In an embodiment, the means are comprised by a single device. The system in an embodiment comprises (i) a reception unit for obtaining measurement data in step (I) and (ii) a computer unit for processing the data obtained to perform at least the determining step as specified. As the skilled person understands, the processor of the system may e.g. be the processor of the computer unit, or may be a further processor.

Typical means for obtaining measurement data are known in the art. Thus, the reception unit may be any means allowing the processor to correspond with a device comprising measurement data by any means for data transmission. Such data transmission may be achieved by a permanent or temporary physical connection, such as coaxial, fiber, fiber-optic or twisted-pair, 10 BASE-T cables. Typically, however, it may be achieved by a temporary or permanent wireless connection using, e.g., radio waves, such as Wi-Fi, LTE, LTE-advanced or Bluetooth. Also, measurement data may be obtained via a reading device from a permanent memory device, such as a CD, DVD, a USB memory device, an SD card, or the like. The system may comprise a multiplex PCR device, e.g., the processor configured to carry out steps (I) and (II) may be the processor of a multiplex PCR device or system. The system may, however, also comprise a processor as specified herein above, and in addition a multiplex PCR device. The system and/or the multiplex PCR device may comprise a container as specified herein above, and at least one detector for detecting a signal of an indicator in at least a first and a second channel. Appropriate detectors are selected by the skilled person without further ado in dependence of the signal in the channel to be detected and in particular of the indicator. I.e. in case a color channel shall be detected, the detector may be any optical detector deemed appropriate by the skilled person, e.g. a fluorescence detector. The unit detecting the actual signal may be coupled to a further unit adjusting a parameter of a further channel, e.g. a temperature. Thus, in case the multiplex PCR is a multi-temperature channel multiplex PCR, the detector may comprise an adjustable thermostat and a fluorescence detection unit.

In an embodiment, the system further comprises a data storage device. In a further embodiment, said data storage device comprises reference values, e.g. in a reference value data base. Moreover, in an embodiment, the data storage unit is adapted to store measurement data received by the receiving unit and/or output data received from the processor of the system. In an embodiment, the data storage device comprises a computer program comprising instructions which, when the program is executed by the least one processor cause the processor to perform at least step (iv) of the method described herein. In a further embodiment, said computer program further comprises instructions which, when the program is executed by the least one processor cause the processor to control the system to further perform any one of steps (ii) and (iii) of the method described herein. In a further embodiment, the system comprises a processor and a database as well as software which is tangibly embedded to said system and, when running on said system, carries out the steps as described. More typically, the system may also comprise a user interface, such as a screen, which allows for providing the result of the analysis carried out to a user.

Typical systems are those which can be applied without the particular knowledge of a specialized technician, e.g., electronic devices which merely require loading with a sample. The results may be given as output of raw data which need interpretation by a technician. In an embodiment, the output of the device is, however, processed, i.e. evaluated, raw data, the interpretation of which does not require a technician.

The present invention also relates to a use of a first label-1 crosstalk signal and a label-2 signal for determining a first and a second target polynucleotide in a sample.

The invention further discloses and proposes a computer program including computer-executable instructions for performing the method according to the present invention in one or more of the embodiments enclosed herein when the program is executed on a computer or computer network. Specifically, the computer program may be stored on a computer-readable data carrier. Thus, specifically, one, more than one or even all of method steps a) to d) as indicated above may be performed by using a computer or a computer network, in an embodiment by using a computer program.

The invention further discloses and proposes a computer program product having program code means, in order to perform the method according to the present invention in one or more of the embodiments enclosed herein when the program is executed on a computer or computer network. Specifically, the program code means may be stored on a computer-readable data carrier.

Further, the invention discloses and proposes a data carrier having a data structure stored thereon, which, after loading into a computer or computer network, such as into a working memory or main memory of the computer or computer network, may execute the method according to one or more of the embodiments disclosed herein.

The invention further proposes and discloses a computer program product with program code means stored on a machine-readable carrier, in order to perform the method according to one or more of the embodiments disclosed herein, when the program is executed on a computer or computer network. As used herein, a computer program product refers to the program as a tradable product. The product may generally exist in an arbitrary format, such as in a paper format, or on a computer-readable data carrier. Specifically, the computer program product may be distributed over a data network.

Finally, the invention proposes and discloses a modulated data signal which contains instructions readable by a computer system or computer network, for performing the method according to one or more of the embodiments disclosed herein.

In an embodiment, referring to the computer-implemented aspects of the invention, one or more of the method steps or even all of the method steps of the method according to one or more of the embodiments disclosed herein may be performed by using a computer or computer network. Thus, generally, any of the method steps including provision and/or manipulation of data may be performed by using a computer or computer network. Generally, these method steps may include any of the method steps, typically except for method steps requiring manual work, such as providing the samples and/or certain aspects of performing the actual measurements.

Specifically, the present invention further discloses:
- A computer or computer network comprising at least one processor, wherein the processor is adapted to perform the method according to one of the embodiments described in this description,
- a computer loadable data structure that is adapted to perform the method according to one of the embodiments described in this description while the data structure is being executed on a computer,
- a computer program, wherein the computer program is adapted to perform the method according to one of the embodiments described in this description while the program is being executed on a computer,
- a computer program comprising program means for performing the method according to one of the embodiments described in this description while the computer program is being executed on a computer or on a computer network,
- a computer program comprising program means according to the preceding embodiment, wherein the program means are stored on a storage medium readable to a computer,
- a storage medium, wherein a data structure is stored on the storage medium and wherein the data structure is adapted to perform the method according to one of the embodiments described in this description after having been loaded into a main and/or working storage of a computer or of a computer network, and
- a computer program product having program code means, wherein the program code means can be stored or are stored on a storage medium, for performing the method according to one of the embodiments described in this description, if the program code means are executed on a computer or on a computer network.

Summarizing the findings of the present invention, the following embodiments are particularly envisaged:
Embodiment 1: A method for performing a multiplex PCR to determine at least a first target polynucleotide and a second target polynucleotide in a sample, comprising
   (i) contacting said sample in a single container with at least a first probe oligonucleotide for the first target polynucleotide and with a second probe oligonucleotide for the second target polynucleotide;
      wherein said first probe oligonucleotide is labeled with a first label (label-1) emitting a label-1 signal in a first channel, a first label-1 crosstalk signal in a second channel, and a second label-1 crosstalk signal in a third channel, and
      wherein said second probe oligonucleotide is labeled with a second label (label-2) emitting a label-2 signal in said third channel;
   (ii) amplifying said target polynucleotides by PCR;
   (iii) measuring a multitude of values in the second channel and the third channel over at least part of the amplification in step (ii); and
   (iv) determining the first target polynucleotide based on the values measured in step (iii).
Embodiment 2: The method of embodiment 1, wherein said multiplex PCR is a multi-temperature channel multiplex PCR and wherein said first channel is a first temperature, said second channel is a second temperature, and wherein said third channel is a third temperature.
Embodiment 3: The method of embodiment 2, wherein said first temperature is lower than the second temperature and wherein said second temperature is lower than the third temperature.
Embodiment 4: The method of embodiment 2 or 3, wherein the temperature difference between the first channel and the second channel is at least 10°C, in an embodiment is at least 15°C, and/or the temperature difference between the second channel and the third channel is at least 10°C, in an embodiment is at least 15°C.
Embodiment 5: The method of any one of embodiments 2 to 4, wherein the temperature of the first channel is more than 40°C and up to 65°C, wherein the temperature of the second channel is more than 65°C and up to 80°C, and/or wherein the temperature of the third channel is more than 80°C and up to 100°C.
Embodiment 6: The method of any one of embodiments 1 to 5, wherein said first target polynucleotide is non-identical to said second target polynucleotide.
Embodiment 7: The method of any one of embodiments 1 to 6, wherein said first probe oligonucleotide comprises a nucleotide sequence at least partially complementary to the first target polynucleotide (annealing portion of the first probe oligonucleotide) and wherein said second probe oligonucleotide comprises a nucleotide sequence at least partially complementary to the second target polynucleotide (annealing portion of the second probe oligonucleotide). Embodiment 8: The method of any one of embodiments 1 to 7, wherein said first probe oligonucleotide specifically hybridizes within the first target polynucleotide and wherein said second probe oligonucleotide specifically hybridizes within the second target polynucleotide. Embodiment 9: The method of any one of embodiments 1 to 8, wherein said first probe oligonucleotide comprises a first indicator and wherein said second probe oligonucleotide comprises a second indicator.
Embodiment 10: The method of any one of embodiments 2 to 9, wherein said first indicator and said second indicator are identical.
Embodiment 11: The method of any one of embodiments 1 to 10, wherein said first indicator and/or said second indicator is/are Cy5.5.
Embodiment 12: The method of any one of embodiments 9 to 11, wherein said first probe oligonucleotide further comprises an annealing portion quencher capable of quenching the detectable signal generated by the first indicator and/or wherein said second probe oligonucleotide further comprises an annealing portion quencher capable of quenching the detectable signal generated by the second indicator.
Embodiment 13: The method of embodiment 12, wherein the first indicator and the annealing portion quencher of the first probe oligonucleotide are intervened by a nuclease susceptible cleavage site and/or wherein the second indicator and the annealing portion quencher of the second probe oligonucleotide are intervened by a nuclease susceptible cleavage site.
Embodiment 14: The method of any one of embodiments 1 to 13, wherein said first probe oligonucleotide further comprises a first tag portion comprising an at least partially double-stranded oligonucleotide of which one strand is covalently connected to the annealing portion and/or wherein said second probe oligonucleotide further comprises a second tag portion comprising an at least partially double-stranded oligonucleotide of which one strand is covalently connected to the annealing portion.
Embodiment 15: The method of embodiment 14, wherein the indicator of the first probe oligonucleotide is comprised in said strand covalently connected to the annealing portion and/or wherein the indicator of the second probe oligonucleotide is comprised in said strand covalently connected to the annealing portion.
Embodiment 16: The method of embodiment 14 or 15, wherein said tag portion of the first polynucleotide further comprises in the strand not covalently connected to the annealing portion a tag portion quencher capable of quenching the detectable signal generated by the first indicator and/or wherein said tag portion of the second polynucleotide further comprises in the strand not covalently to the annealing portion a tag portion quencher capable of quenching the detectable signal generated by the second indicator.
Embodiment 17: The method of embodiment 16, wherein the first indicator, the tag portion quencher, and the annealing portion quencher of the first probe oligonucleotide together form the first label, and/or wherein the second indicator, the tag portion quencher, and the annealing portion quencher of the second probe oligonucleotide together form the second label. Embodiment 18: The method of any one of embodiments 14 to 17, wherein the nucleotide sequences comprised in said first and second tag portions are non-complementary to the first and second target sequences.
Embodiment 19: The method of any one of embodiments 14 to 18, wherein the tag portion of the first probe oligonucleotide does not specifically hybridize to any target polynucleotide and/or wherein the tag portion of the second probe oligonucleotide does not specifically hybridize to any target polynucleotide.
Embodiment 20: The method of any one of embodiments 14 to 19, wherein the tag portion is attached to the 5' terminus of the annealing portion or wherein the tag portion is attached to the 3 ' terminus of the annealing portion in said first probe oligonucleotide and/or in said second probe oligonucleotide.
Embodiment 21: The method of any one of embodiments 1 to 20, wherein said first label is a first interactive dual label and/or wherein said second label is a second interactive dual label.
Embodiment 22: The method of embodiment 16 to 21, wherein said annealing portion quencher is identical to said tag portion quencher in said first probe oligonucleotide and/or said second probe oligonucleotide.
Embodiment 23: The method of any one of embodiments 12 to 22, wherein said annealing portion quencher of the first probe oligonucleotide is identical to the annealing portion quencher of the second probe oligonucleotide.
Embodiment 24: The method of any one of embodiments 16 to 23, wherein said tag portion quencher of the first probe oligonucleotide is identical to the tag portion quencher of the second probe oligonucleotide.
Embodiment 25: The method of any one of embodiments 16 to 24, wherein said tag portion quencher is BHQ-2 or BHQ-3.
Embodiment 26: The method of any one of embodiments 12 to 25, wherein said annealing portion quencher is BHQ or BHQ-3.
Embodiment 27: The method of any one of embodiments 16 to 26, wherein the tag portion comprises a modification such that it is not capable of being extended by a nucleic acid polymerase.
Embodiment 28: The method of any one of embodiments 1 to 27, wherein the first probe oligonucleotide and/or the second probe oligonucleotide contain(s) at least one nucleotide modification.
Embodiment 29: The method of embodiment 28, wherein the at least one nucleotide modification is independently selected from the group consisting of a Locked Nucleic Acid (LNA) nucleotide, a Peptide Nucleic Acid (PNA) nucleotide, a Bridged Nucleic Acid (BNA) nucleotide, a 2'-O alkyl substitution, an L-enantiomeric nucleotide, and combinations thereof.
Embodiment 30: The method of any one of embodiments 1 to 29, wherein step (iv) comprises determining the first target polynucleotide based on the values of the first label-1 cross-talk signal and determining the second target polynucleotide based on the values of the label-2 signal and the values of the second label-1 crosstalk signal determined in step (iii).
Embodiment 31: The method of any one of embodiments 1 to 30, wherein step (iv) comprises linearly compensating the second label-1 crosstalk signal based on the label-1 signal when determining the second target polynucleotide.
Embodiment 32: The method of any one of embodiments 1 to 31, wherein the first label-1 crosstalk signal cannot be linearly compensated in step (iv).
Embodiment 33: The method of any one of embodiments 1 to 32, wherein the second label-1 crosstalk signal can be linearly compensated in step (iii).
Embodiment 34: A system comprising at least one processor configured to
   (I) obtain measurement data from step (iii) of a method according to any one of embodiments 1 to 33; and
   (II) perform at least determining step (iv) of said method.
Embodiment 35: The system of embodiment 34, further comprising at least one of: a PCR device; a container; and/or at least one detector for detecting an indicator in at least a first and a second channel.
Embodiment 36: The system of embodiment 34 or 35, further comprising at least one data storage device.
Embodiment 37: The system of embodiment 36, wherein said data storage device comprises a computer program comprising instructions which, when the program is executed by the least one processor cause the processor to perform at least step (iv) of the method according to any one of embodiments 1 to 33.
Embodiment 38: The system of embodiment 37, wherein the computer program further comprises instructions which, when the program is executed by the least one processor cause the processor to control the system to further perform any one of steps (ii) and (iii) of the method according to any one of embodiments 1 to 33.
Embodiment 39: A computer program comprising instructions which, when the program is executed by at least one processor of the system according to any one of embodiments 34 to 38 cause the processor to perform at least step (iv) of the method according to any one of embodiments 1 to 33.
Embodiment 40: A computer-readable storage medium, specifically a non-transient computer-readable storage medium, comprising instructions which, when the instructions are executed by at least one processor of the system according to any one of embodiments 34 to 37 cause the processor to perform at least step (iv) of the method according to any one of embodiments 1 to 33.
Embodiment 41: Use of a first label-1 crosstalk signal and a label-2 signal for determining a first and a second target polynucleotide in a sample.
Embodiment 42: The use of embodiment 41, wherein said use has a feature of any of the preceding embodiments.
Embodiment 43: The method of any one of embodiments 1 to 33, wherein the improvement comprises linearly compensating the second label-1 crosstalk signal based on the label-1 signal when determining the second target polynucleotide, thereby enabling the use of the multitude of values in the second channel and the third channel determined in step (iii) for determining both said first target polynucleotide and said second target polynucleotide in step (iv).

All references cited in this specification are herewith incorporated by reference with respect to their entire disclosure content and the disclosure content specifically mentioned in this specification.

### Figure Legends

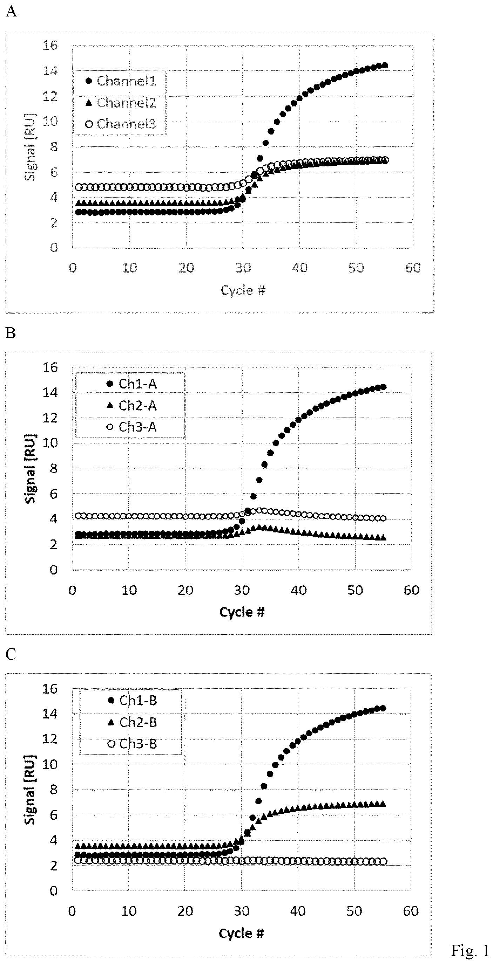

Fig. 1: Exemplary signal intensity (arbitrary units, RU) over PCR cycle number in case channel 1 (low temperature channel) is positive; (A) raw data, (B) after standard linear compensation, note the incomplete compensation in the range of cycle numbers 30 to 40; (C) after compensation according to the invention.

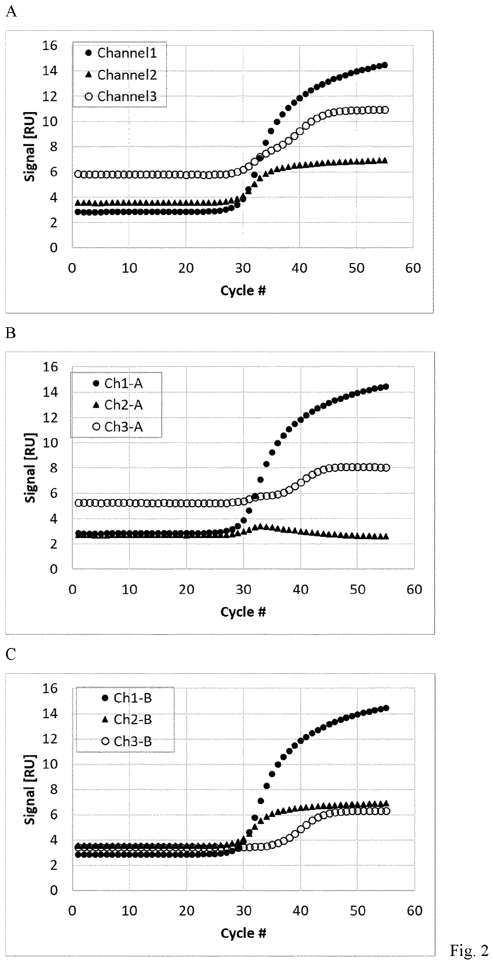

Fig. 2: Exemplary signal intensity (arbitrary units, RU) over PCR cycle number in case channels 1 and 3 (low temperature channel and high temperature channel) are positive; (A) raw data, (B) after standard linear compensation, note the incomplete compensation in the range of cycle numbers 30 to 40; (C) after compensation according to the invention.

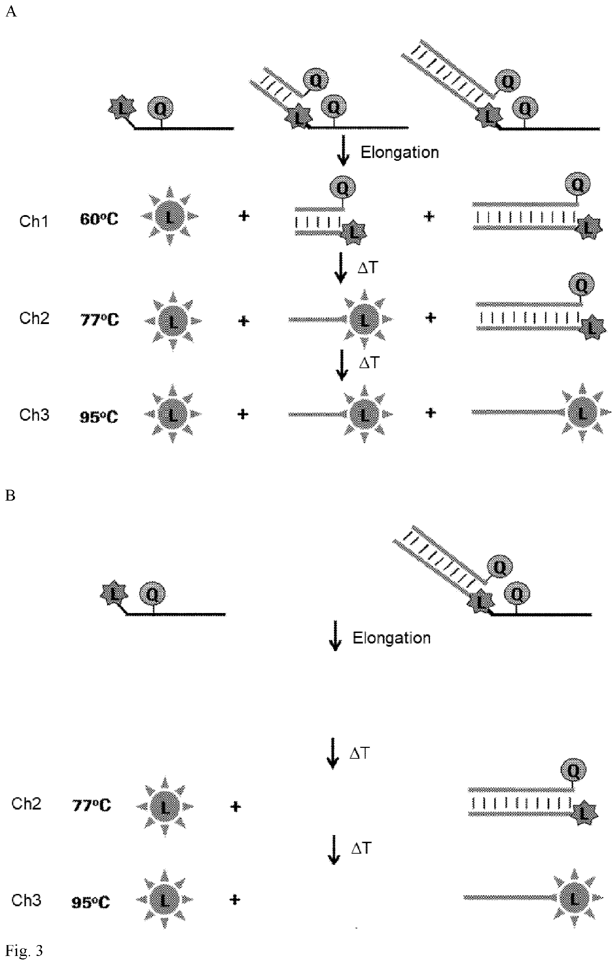

Fig. 3: Schematic representation of a multi-temperature channel PCR; (A) standard proceeding, Figure modified from US 2018/0073064 A1: if target polynucleotides are present, the indicator becomes separated from the quencher in the low temperature channel (Ch1) by 5'->3' exonuclease activity of the thermostable DNA polymerase during elongation; also, tag portions of probe oligonucleotides become separated from annealing portions and their quenchers; after temperature increase (ΔT) to 77°C, the tag portion of the middle temperature channel (Ch2) probe oligonucleotide dissociates, but not the tag portion of the high temperature channel (Ch3) probe oligonucleotide, which only dissociates after a further temperature increase to 95°C; if the crosstalk signals between the channels cannot be compensated, only one channel can be used; (B) Proceeding according to the invention: As in (A), but omitting the Ch2 probe oligonucleotide and measuring the signal of the Ch1 probe oligonucleotide as its crosstalk signal in Ch2; since the crosstalk signal in Ch3 can be linearly compensated, Ch3 can be used for an additional measurement, e.g. of an additional target polynucleotide; Q: quencher, L: indicator.

The following Examples shall merely illustrate the invention. They shall not be construed, whatsoever, to limit the scope of the invention.

### Example 1: Multi-temperature channel multiplex PCR, Ch1 positive

Multi-temperature channel multiplex PCR was performed essentially as described in US 2018/0073064 A1, using Cy5.5 as the indicator in all temperature channels and BHQ as a quencher. PCT was performed for 55 cycles under standard conditions, and fluorescence was measured in three temperature channels (60°C, 77°C, and 95°C).

Results are shown in Fig. 1 for the case that only channel 1 (low-temperature channel) is positive, Fig. 1A) showing raw data, Fig. 1B) showing the result of standard compensation, and Fig. 1C) showing results after compensation according to the invention. As will be appreciated, compensation according to the invention allows for complete compensation of channel cross-talk, which standard compensation does not.

### Example 2: Multi-temperature channel multiplex PCR, Ch1 and Ch3 positive

Fig. 2 shows results analogous to those of Example 1, except that channels 1 and 3 (low temperature and high temperature channels) are positive.

As a result, compensation as described herein enables that low temperature channel 1 (measured via its cross-talk signal in the middle-temperature channel 2) can be used in combination with the high-temperature channel 3, thus providing one additional usable channel.

### Example 3 (Fig. 3)

Fig. 3 shows schematically the proceeding in multi-temperature channel multiplex PCR, A) as described in US 2018/0073064 A1, and B) according to the instant invention. For details, cf. Figure legend.

## Claims

1. A method for performing a multiplex PCR to determine at least a first target polynucleotide and a second target polynucleotide in a sample, comprising
(i) contacting said sample in a single container with at least a first probe oligonucleotide for the first target polynucleotide and with a second probe oligonucleotide for the second target polynucleotide;
wherein said first probe oligonucleotide is labeled with a first label (label-1) emitting a label-1 signal in a first channel, a first label-1 crosstalk signal in a second channel, and a second label-1 crosstalk signal in a third channel, and
wherein said second probe oligonucleotide is labeled with a second label (label-2) emitting a label-2 signal in said third channel;
(ii) amplifying said target polynucleotides by PCR;
(iii) measuring a multitude of values in the second channel and the third channel over at least part of the amplification in step (ii); and
(iv) determining the first target polynucleotide based on the values measured in step (iii).

2. The method of claim 1, wherein said multiplex PCR is a multi-temperature channel multiplex PCR and wherein said first channel is a first temperature, said second channel is a second temperature, and wherein said third channel is a third temperature.

3. The method of claim 2, wherein the temperature of the first channel is more than 40°C and up to 65°C, wherein the temperature of the second channel is more than 65°C and up to 80°C, and/or wherein the temperature of the third channel is more than 80°C and up to 100°C.

4. The method of any one of claims 1 to 3, wherein said first probe oligonucleotide comprises a nucleotide sequence at least partially complementary to the first target polynucleotide (annealing portion of the first probe oligonucleotide) and wherein said second probe oligonucleotide comprises a nucleotide sequence at least partially complementary to the second target polynucleotide (annealing portion of the second probe oligonucleotide).

5. The method of any one of claims 1 to 4, wherein said first probe oligonucleotide comprises a first indicator and wherein said second probe oligonucleotide comprises a second indicator, in an embodiment wherein said first indicator and said second indicator are identical, in a further embodiment wherein said first indicator and/or said second indicator is/are Cy5.5.

6. The method of claim 5, wherein said first probe oligonucleotide further comprises an annealing portion quencher capable of quenching the detectable signal generated by the first indicator and/or wherein said second probe oligonucleotide further comprises an annealing portion quencher capable of quenching the detectable signal generated by the second indicator.

7. The method of claim 6, wherein the first indicator and the annealing portion quencher of the first probe oligonucleotide are intervened by a nuclease susceptible cleavage site and/or wherein the second indicator and the annealing portion quencher of the second probe oligonucleotide are intervened by a nuclease susceptible cleavage site.

8. The method of any one of claims 1 to 7, wherein said first probe oligonucleotide further comprises a first tag portion comprising an at least partially double-stranded oligonucleotide of which one strand is covalently connected to the annealing portion; and/or wherein said second probe oligonucleotide further comprises a second tag portion comprising an at least partially double-stranded oligonucleotide of which one strand is covalently connected to the annealing portion.

9. The method of claim 8, wherein the indicator of the first probe oligonucleotide is comprised in said strand covalently connected to the annealing portion and/or wherein the indicator of the second probe oligonucleotide is comprised in said strand covalently connected to the annealing portion.

10. The method of claim 8 or 9, wherein said tag portion of the first polynucleotide further comprises in the strand not covalently connected to the annealing portion a tag portion quencher capable of quenching the detectable signal generated by the first indicator and/or wherein said tag portion of the second polynucleotide further comprises in the strand not covalently to the annealing portion a tag portion quencher capable of quenching the detectable signal generated by the second indicator.

11. The method of claim 10, wherein the first indicator, the tag portion quencher, and the annealing portion quencher of the first probe oligonucleotide together form the first label, and/or wherein the second indicator, the tag portion quencher, and the annealing portion quencher of the second probe oligonucleotide together form the second label.

12. The method of any one of claims 1 to 11, wherein step (iv) comprises linearly compensating the second label-1 crosstalk signal based on the label-1 signal when determining the second target polynucleotide.

13. A system comprising at least one processor configured to
(I) obtain measurement data from step (iii) of a method according to any one of claims 1 to 12; and
(II) perform at least determining step (iv) of said method.

14. A computer-readable storage medium, specifically a non-transient computer-readable storage medium, comprising instructions which, when the instructions are executed by the at least one processor of the system according to claim 13, cause the processor to perform at least step (iv) of the method according to any one of claims 1 to 12.

15. Use of a first label-1 crosstalk signal and a label-2 signal for determining a first and a second target polynucleotide in a sample.
